# EUROPEAN PATENT SPECIFICATION

(11) **EP 1 848 458 B1**
(45) Date of publication and mention of the grant of the patent: **04.11.2009**
(21) Application number: 06706962.5
(22) Date of filing: 15.02.2006
(51) Int. Cl.: A61K 47/48, C07K 1/107, C07K 16/46

(54) **USE OF ACTIVATED POLYMERS FOR SEPARATION OF PROTEIN AND POLYPEPTIDE MULTIMERS**
VERWENDUNG AKTIVIERTER POLYMERE ZUR TRENNUNG VON PROTEIN- UND POLYPEPTID-MULTIMEREN
UTILISATION DE POLYMERES ACTIVES POUR SEPARER DES MULTIMERES PROTEIQUES ET POLYPEPTIDIQUES

(30) Priority: 16.02.2005 EP 05003332
(43) Date of publication of application: 31.10.2007
(73) Proprietor: Micromet AG, 81477 München (DE)
(72) Inventor: WOLF, Andreas, 82131 Gauting (DE); HOFFMANN, Patrick, 83670 Bad Heilbrunn (DE); HEPP, NEE HENCKEL, Julia, CH-8044 Zürich (CH); RAUM, Tobias, 81925 München (DE)
(74) Representative: Dehmel, Albrecht
(86) International application number: PCT/EP2006/001359
(87) International publication number: WO 2006/087178

(56) References cited:
- WO-A-03/000179
- US-A- 5 478 805
- US-A1- 2002 044 921
- FRANCIS G E ET AL: "POLYETHYLENE GLYCOL MODIFICATION: RELEVANCE OF IMPROVED METHODOLOGY TO TUMOUR TARGETING" JOURNAL OF DRUG TARGETING, HARWOOD ACADEMIC PUBLISHERS GMBH, DE, vol. 3, no. 5, 1996, pages 321-340, XP001027078 ISSN: 1061-186X
- VERONESE F M: "Peptide and protein PEGylation - a review of problems and solutions" BIOMATERIALS, ELSEVIER SCIENCE PUBLISHERS BV., BARKING, GB, vol. 22, no. 5, 1 March 2001 (2001-03-01), pages 405-417, XP004227886 ISSN: 0142-9612
- ROBERTS M J ET AL: "Chemistry for peptide and protein PEGylation" ADVANCED DRUG DELIVERY REVIEWS, AMSTERDAM, NL, vol. 54, no. 4, 17 June 2002 (2002-06-17), pages 459-476, XP002293146 ISSN: 0169-409X

## Description

The present invention relates to a use of activated polymers. Specifically, the present invention relates to a use of activated polymers in the field of polypeptide and/or protein preparation.

Following recombinant expression, polypeptide and/or protein molecules often exist in one or more isoforms, that is these molecules often exhibit product heterogeneity. As a particularly well studied example, single chain antibodies, or scFvs, are known to exist following recombinant expression as a mixture of monomeric and multimeric, primarily dimeric, species. The monomeric species results from the covalent or non-covalent association of antibody variable regions on the same polypeptide chain with one another. On the other hand, the dimeric species results from association of first and second polypeptide chains, each comprising, say, two complementary antibody variable regions A and B with one another such that the variable region A of the first polypeptide chain associates with variable region B of the second polypeptide chain, and vice versa. This species is commonly known as a diabody (Hudson et al. (1999) J. Immunol. Met. 231, 177-189).

Where polypeptides are intended for later therapeutic use, such product heterogeneity is generally undesirable, as heterogeneous products often exhibit distinct biological activities or pharmacokinetic properties. In developing a polypeptide therapeutic, it is important to be able to predict how this polypeptide will act *in vivo* (i.e. its qualitative mode of action) as well as the magnitude of this biological activity (i.e. its quantitative efficacy and distribution in the body). Such predictions are often difficult to confidently make for heterogeneous products (Moore et al. (1999) Biochemistry. 38, 13960-13967).

However, it can also be problematic to create a homogeneous product which remains so. This is because polypeptide heterogeneity is often the result of a thermodynamic equilibrium between monomeric and multimeric polypeptide; following removal of one of the species, equilibrium is re-established between both species (Lee et al. (2002) J. Mol. Biol. 320,107-127). This applies for both the fraction removed, as well as the remaining fraction so that at some time after chromatographic separation each fraction will exhibit approximately the same monomer:multimer ratio, this ratio being the result of isomeric equilibration between both, or all polypeptide species. Simple chromatographic removal of one of the polypeptide species, then, often represents only a transient solution to the problem of product heterogeneity, as there often exists a natural thermodynamic drive to heterogeneous product. This problem can be circumvented by converting a desired purified product into a form in which thermodynamic equilibration between isoforms can no longer take place, for example into a lyophilisate. This lyophilisate is then reconstituted immediately prior to administration at the point of care, so that undesired thermodynamic equilibration and a concomitant increase in product heterogeneity is eliminated or at least kept to an absolute minimum. However, separation of the undesired isoform from a heterogeneous mixture often results in significant loss of polypeptide product.

Independently of the problems of polypeptide heterogeneity, the developer of therapeutic polypeptides is often faced with the need to modulate the pharmacokinetic and/or immunogenic properties of the polypeptide intended for administration. For example, the polypeptide intended for therapy may be eliminated from patient serum too quickly to elicit any therapeutic effect, a problem which generally becomes more intractable the lower the molecular weight of the polypeptide is. The rate of elimination of a therapeutic polypeptide from the serum of a patient may be undesirably accelerated if the polypeptide triggers an immunogenic response, that is if the patient's immune system mounts an immune response to the foreign material. For each of these reasons, it is often desirable to derivatize a polypeptide intended for administration to a patient such that serum half life is extended and the immunogenicity of the polypeptide is reduced, the former resulting at least in part from the latter.

These objectives have been met by conjugation of extended organic polymers to therapeutic polypeptides. To cite one example in the literature, conjugation of polypeptides with polyethylene glycol ("PEG") has been used for this purpose (Roberts et al. (2002) Adv. Drug Delivers Rev. 54, 459-476). Conjugation with extended organic polymers increases the effective molecular weight of polypeptides while at the same time shielding them from recognition by the immune system - each of which has the effect that the serum half-life of the polypeptide is advantageously extended.

The developer of therapeutic polypeptides is therefore often faced with the twofold challenge of generating a homogeneous product while improving this product's pharmacokinetic and/or immunogenic properties. Combined, these considerations necessitate numerous sequential isolation, purification and conjugation steps, each of which results in loss of product and time and, in general, an added complexity and cost over the entire production process. It is therefore an aim of the invention to provide an alternative, more concerted way of addressing the problems mentioned above.

The inventors of the present invention have now surprisingly found that conjugation of polypeptides with extended polymers results not only in the advantages already recognized for such polymers (i.e. improving a product's pharmacokinetic and/or immunogenic properties), but also in the separation of multimeric polypeptide into conjugated monomeric polypeptide in a permanent fashion.

Accordingly, one aspect of the present invention pertains to the use of an activated polymer to separate a non-covalently associated polypeptide multimer comprising multiple polypeptide subunits into said multiple polypeptide subunits in accordance with the attached claims.

According to this aspect of the invention, a polymer, preferably an organic polymer, is used in activated form. By "activated" is meant any form of the polymer comprising a chemical moiety by which the polymer may be covalently bound to a polypeptide subunit in the non-covalently associated polypeptide multimer. Since a polymer comprising such a moiety will retain this moiety following covalent binding to the polypeptide subunit, the term "activated polymer" as used herein refers to the polymer both prior to as well as following coupling to the polypeptide subunit, i.e. an activated polymer which has already formed a covalent bond with a polypeptide subunit will still be referred to as an "activated polymer". Preferably, the chemical moiety for covalently binding to the polypeptide will react under physiological conditions or near-physiological conditions, or at least under conditions which will not be harmful to the polypeptide subunits of the non-covalently associated polypeptide multimer.

The term "non-covalently associated polypeptide multimer" is to be understood as encompassing any polypeptide species comprising at least two polypeptide chains which are separable from one another without breaking any covalent chemical bonds. The association may be of an ordered nature, for example of the type seen between two polypeptides which are sterically and/or electrostatically complementary to one another. As an example of such an ordered association, one may imagine a polypeptide homodimer of the sort described above in the context of dimeric single chain antibodies (i.e. "diabodies"). Alternatively, the association may be of a disordered nature, for example of the type observed in a polypeptide precipitate in which individual polypeptide chains agglomerate and become insoluble in aqueous solution. As an example of such a disordered association, one may imagine insoluble inclusion bodies resulting from recombinant expression of polypeptides.

The inventive use is, however, independent of whether the polypeptide multimer is associated in an ordered or in a disordered manner. Upon covalent reaction between at least one, preferably each of the multiple polypeptide subunits of the non-covalently associated polypeptide multimer with at least one molecule of the activated polymer as described above, these multiple polypeptide subunits become separated from one another along the lines of their mutual non-covalent association.

The term "separate" (meant as a verb) is to be understood as the act of introducing a sufficient distance, in solution, between two previously non-covalently associated polypeptide subunits such that there no longer exist any, or any significant attractive molecular interactions between these two subunits. Molecular interactions which may exist between two non-covalently associated polypeptide subunits prior to separation may for example include one or more of hydrogen bonding interactions, Van der Waals interactions, overlap of delocalized pi-orbitals, hydrophobic interactions and electrostatic/ionic interactions. Following reaction with the activated polymer, the distance between each of the two polypeptide subunits, for reasons elaborated hereinbelow, increases such that the overall attractive forces between these two polypeptide subunits decreases to zero or at least becomes vanishingly small.

The mention of "two polypeptide subunits" should not be understood restrictively, but rather as illustrative of the effected separation of any two given non-covalently associated polypeptide subunits within a non-covalently associated polypeptide multimer. The inventive use is therefore applicable to non-covalently associated polypeptide multimers comprising as few as only two non-covalently associated polypeptide subunits, as well as to non-covalently associated polypeptide multimers comprising two, or even more than two, that is, three, four, five, six, or even many non-covalently associated polypeptide subunits. In the latter case, each of these many polypeptide subunits will be non-covalently associated to one or more other polypeptide subunits; the separation process described in the previous paragraph is to be understood as illustrative of the inventive use's effect at a given interface between any two respective polypeptide subunits. Seen from the standpoint of a single non-covalently associated polypeptide multimer, then, the process of separating this multimer into its constituent multiple polypeptide subunits will entail many such molecular separations occurring sequentially and/or simultaneously between each of the subunit-subunit interfaces comprised within the non-covalently associated polypeptide multimer.

The inventive use entails several advantages. Most prominently, in the realization that an activated polymer can be used not only for improvement of the pharmacokinetic and/or immunogenic properties of a therapeutic polypeptide, but also to render an isomerically heterogeneous polypeptide mixture homogeneous in monomer (i.e. in a single defined species), the inventors have achieved a level of concertedness not previously seen in the production of polypeptides intended for therapeutic use. Not only can the pharmacokinetic and/or immunogenic properties of polypeptides intended for administration be improved in the known way by conjugation to polymer, but this conversion to conjugate may be performed without first having to separate a desired monomeric polypeptide isomer from its various multimeric species. This alone implies a streamlining of the overall production of therapeutic polypeptides, leading as it does in one process step to a result previously obtainable only by performance of multiple process steps. However, the inventive use has a further effect which makes it especially amenable to the production of therapeutic polypeptides. Conjugation to polymer also appears to prevent the undesirable reestablishment of thermodynamic equilibrium between monomeric and multimeric polypeptide species. That is to say, once each polypeptide subunit is separated out of the non-covalently associated polypeptide multimer, this subunit will tend to stay separated and will not reassociate with other subunits to reform a new polypeptide multimer. The inventive use, then, allows not only the establishment but also the maintenance of a desirable product homogeneity in monomeric polypeptide.

In short, the inventive use enables the procurement in a single step of homogeneous, polypeptide conjugated to polymer, which polypeptide-polymer conjugate can then be further purified for therapeutic use.

Several advantageous scenarios are imaginable for application of the inventive use. In the first, one is in possession of a homogeneous or almost homogeneous non-covalently associated polypeptide multimer for which the isomeric equilibrium lies far to the side of the multimer, but the monomeric species is desired in a form conjugated to polymer. In this case, one can imagine the following reaction (in which the multimer is a homodimer comprising two monomeric subunits for purposes of illustration, "AP" represents activated polymer, and "SU" represents a monomeric subunit):

SU·SU + 2 AP --------------> 2 (SU-AP)

Alternatively, one may be in possession of an isomerically heterogeneous polypeptide mixture comprising an equilibrium ratio of (here, again) homodimeric and monomeric polypeptide, wherein the dimer comprises two identical subunits, and the monomer comprises a polypeptide identical to each of the two subunits in the dimer (abbreviations as above):

SU + SU·SU + 3 AP --------------> 3 (SU-AP)

In each of the two non-exhaustive scenarios depicted above, the inventive use of activated polymer results, in one step, in a product homogeneous in monomer and properly conjugated to polymer for later use as a therapeutic. Further scenarios are imaginable, and these are described in further detail below.

Without being bound by theory, the inventors believe that the advantageous effect of the inventive use may be related to the kinetic fluctuations which take place in any polypeptide solution over time. Specifically, a given polypeptide structure is known to continually transition between different conformational states, i.e. substructures. The rapidity of this transitioning depends on a number of factors, among them the particular amino acid sequence of the polypeptide and the temperature of the medium. As an example in the field of single chain antibody technology, two intramolecularly associated antibody variable regions mutually connected by a polypeptide linking sequence are known to continually open and close in solution; one speaks of "molecular breathing." In the specific context of two non-covalently associated polypeptide subunits, such "breathing" occurs not between two polypeptide regions located on the same polypeptide chain, but between two distinct but non-covalently associated polypeptide chains belonging to two distinct polypeptide subunits. In the event, as in the inventive use, that at least one of these two polypeptide subunits has become conjugated to at least one molecule of activated polymer, part of the unstructured polymer can slip between the two polypeptide subunits when these transition between two conformations and, in doing so, move further apart from one another. In the region where the polymer structure has become interposed between the adjacent polypeptide subunits, the latter cannot re-approach one another in the region of polymer interposition, though they still remain largely associated at other regions between which no polymer has become interposed. In the next conformational transition, when the still associated portions of the two polypeptide subunits again move apart from one another, the polymer already partially inserted slips deeper in between the two polypeptide subunits, so that even more of the surfaces of the two polypeptide subunits are prevented from re-associating, and so on. The inventors believe that progressive interposition of the polymer as a sort of wedge between two polypeptide subunits of the polypeptide multimer serves, in time, to fray the polypeptide multimer into its constituent polypeptide subunits, each of which in the end is or becomes conjugated to at least one activated polymer of its own.

According to one embodiment of the invention, the activated polymer has a molecular weight of at least 3,000 g/mol and comprises from 25 to 70 wt.% polar atoms. The molecular weight of the activated polymer required to achieve the advantageous effect as set out above will generally vary directly with the size of the hydrophobic portions within the polypeptide subunit by way of which multimerization occurs. As the size of such hydrophobic portions will not vary directly with the size of the polypeptide subunits within the non-covalently associated polypeptide multimer, it is not readily possible to predict *a priori* exactly which molecular weight activated polymer should be used to yield optimal results, given prior knowledge of molecular weight of the polypeptide subunits to be separated. However, preferred embodiments of the invention envision the use of activated polymer having a molecular weight of 3,500 g/mol, 5,000 g/mol, 20,000 g/mol or 40,000 g/mol. Here, it must be understood that the molecular weight values given herein represent average molecular weight values, as is common in the field of polymer chemistry. That is to say that the molecular weight values given herein represent the most frequently encountered molecular weight in a Gaussian distribution of many molecular weights within a sample of activated polymer. As such, the indication of a particular value for molecular weight herein does not exclude the scenario that within a sample of activated polymer, polymer molecules exist with molecular weights both greater and less than the molecular weight value indicated.

Preferred embodiments of the invention envision using an activated polymer comprising from 27 to 60 wt.% polar atoms, in particular 32 to 45 wt.% polar atoms, from 35 to 38 wt.% polar atoms; from 36 to 37 wt.% polar atoms; from 27 to 28 wt.% polar atoms; from 48 to 50 wt.% polar atoms; or from 54 to 56 wt.% polar atoms. Activated polymer with these ranges of polar atom content will generally exhibit the characteristics believed to be responsible for the observed effect.

The term "polar atoms" is to be understood as denoting atoms which enter into hydrogen bonded interactions with water molecules in aqueous solution and which therefore contribute to a polymer exhibiting properties generally classified by those of skill in the art as "hydrophilic". Predominant members of this class of atoms include oxygen, sulfur, fluorine, chlorine, phosphorous, and nitrogen. One of skill in the art will understand that one is generally limited in one's choice of polar atoms by which atoms will also be amenable to inclusion in a therapeutic molecule intended for administration to a patient.

In light of the above mechanism proposed, in which the polypeptide multimer transitioning between different sub-conformations is frayed into its constituent polypeptide subunits by gradual interposition of activated polymer, it can be understood why activated polymers with the above content(s) of polar atoms, i.e. activated polymers which are highly hydrophilic, would be especially well suited to separating the polypeptide subunits of a polypeptide multimer. In aqueous medium, polypeptide subunits of a non-covalently associated polypeptide multimer usually associate with one another at hydrophobic interfaces (Bahadur et al. (2004) J. Mol. Biol. 336, 943-955). Gradual insertion of the activated polymer between such interfaces changes the internal environment between the facing surfaces of the two polypeptide subunits in a fundamental way: the previously hydrophobic environment between the polypeptide subunits becomes increasingly hydrophilic due to the presence of the hydrophilic polymer structure. In such a scenario, the hydrophobic faces of the two polypeptide subunits can no longer interact, and the tendency for these polypeptide subunits to associate is greatly reduced or lost altogether.

According to a further embodiment of the invention, each polypeptide subunit comprised within the non-covalently associated polypeptide multimer comprises a single polypeptide chain and/or a group of at least two single polypeptide chains, wherein the at least two single polypeptide chains are covalently bound to one another to form the group. In the case that each polypeptide subunit comprises a single polypeptide chain, the non-covalently associated polypeptide multimer may be understood as a collection of two or more distinct polypeptide chains which are non-covalently associated with one another. As an example, one may imagine such a non-covalently associated polypeptide multimer as a "diabody" as discussed hereinabove. In such a case, the use of the invention would separate the two individual scFv polypeptide chains from one another such that two distinct scFv molecules are generated, each being covalently bound to at least one molecule of activated polymer.

According to an especially preferred embodiment of the invention, the polypeptide subunit comprises a single polypeptide chain and the single polypeptide chain is a single chain antibody, i.e. an scFv molecule. This embodiment of the invention, then, results in multiple distinct scFv molecules, each covalently bound to at least one molecule, preferably to one molecule of activated polymer, where these scFv molecules were previously non-covalently associated in a non-covalently associated polypeptide multimer.

According to another especially preferred embodiment of the invention, the polypeptide subunit comprises only one variable region of an antibody, such as a variable region of an antibody which is capable of specifically binding to antigen alone, i.e. without being paired with another antibody variable region. Here, then, the non-covalently associated polypeptide multimer comprises many non-covalently associated antibody variable domains, each of which is independently capable of binding antigen, i.e. each of which is a "single domain antibody".

In the case that each polypeptide subunit comprises a group of at least two single polypeptide chains, the inventive use will separate the non-covalently associated polypeptide multimer into at least two groups of single polypeptide chains (i.e. two subunits), wherein each group comprises at least two single polypeptide chains, each of which is covalently bound to at least one other single polypeptide chain within the same subunit. Such covalent attachment will most commonly take the form of disulfide linkages between cysteine residues on two respective polypeptide chains. As an example of a polypeptide subunit which comprises at least two covalently bound single polypeptide chains, one may imagine a Fab molecule comprising an antibody heavy chain and an antibody light chain, wherein the antibody heavy and light chains are disulfide bonded to one another. Here, a non-covalently associated polypeptide multimer comprising multiple Fab molecules non-covalently associated with one another can be separated by the inventive use into multiple distinct Fab molecules, each covalently bound to at least one molecule of activated polymer.

As another example of a polypeptide subunit comprising a group of at least two covalently-bound single polypeptide chains, one can imagine a full antibody molecule, i.e. an IgG molecule comprising two polypeptide heavy chains and two polypeptide light chains, each light chain being covalently, i.e. disulfide-bound to one heavy chain, and the two heavy polypeptide chains being disulfide-bound to one another. Here, a non-covalently associated polypeptide multimer comprising multiple IgG molecules non-covalently associated with one another can be separated by the inventive use into multiple distinct IgG molecules, each covalently bound to at least one molecule of activated polymer.

According to a further embodiment, the non-covalently associated polypeptide multimer may comprise polypeptide subunits of two different types: one type comprising a single polypeptide chain as explained above, and the other type comprising a group of two or more covalently-bound polypeptide chains, as explained above. In such a non-covalently associated polypeptide multimer, a polypeptide subunit comprising a single polypeptide chain may be non-covalently associated with a polypeptide chain of a polypeptide subunit comprising multiple covalently bound polypeptide chains. In this case, the inventive use would result, after separation by conjugation to activated polymer, in a single chain polypeptide subunit covalently bound to at least one molecule, preferably one molecule of activated polymer and, independently, a group of covalently bound single polypeptide chains, the group as a whole being covalently bound to at least one molecule, preferably one molecule of activated polymer.

According to a further embodiment of the invention each polypeptide subunit is covalently bound to the activated polymer via an amino group, a sulfhydryl group, a carboxyl group or a hydroxyl group comprised within the polypeptide subunit. As one of skill in the art knows, most polypeptides will comprise at least one amino, carboxyl and/or hydroxyl group, as these are common amino acid side chain moieties. When the activated polymer used in the inventive use is one which will react covalently with an amino, carboxyl and/or hydroxyl group, it is therefore likely that the separated polypeptide subunit will be covalently attached to more than one molecule of activated polymer. However, using an activated polymer which will form a covalent bond with a sulfhydryl group in the polypeptide subunits comprised within the polypeptide multimer is especially preferred, since the number of such groups in polypeptide can often be tuned (by incorporation or omission of cysteine residues) such that no more than one activated polymer will be covalently bound to the polypeptide subunit following separation from the non-covalently associated polypeptide multimer. For therapeutic applications, it is often in the interest of an advantageous product homogeneity to limit the number of activated polymers which are attached to a therapeutic polypeptide to a certain number. For the purposes of the present inventive use of activated polymers, it is generally sufficient to limit the number of activated polymers which are finally covalently bound to a polypeptide subunit to one.

In the case that the activated polymer is capable of forming a covalent chemical bond with an amino group comprised within the polypeptide subunit, the activated polymer advantageously comprises a hydroxysuccinimidyl group, a carboxyl group, an epoxide group, a keto group or an aldehyde group. All of these groups are capable of covalently reacting with amine under physiological, or near-physiological conditions. In the case that the activated polymer is capable of forming a covalent chemical bond with a sulfhydryl group comprised within the polypeptide subunit, the activated polymer advantageously comprises a maleimide group, a vinyl sulfone group or a sulfhydryl group, preferably a maleimide group. All of these groups are capable of covalently reacting with sulfhydryl under physiological, or near-physiological conditions. In the case that the activated polymer is capable of forming a covalent chemical bond with a carboxyl group comprised within the polypeptide subunit, the activated polymer advantageously comprises an amino group or a hydroxyl group. Both of these groups are capable of covalently reacting with carboxyl under physiological, or near-physiological conditions. In the case that the activated polymer is capable of forming a covalent chemical bond with a hydroxyl group comprised within the polypeptide subunit, the activated polymer advantageously comprises a carboxyl group, an aldehyde group or a keto group, the carboxyl group being especially preferred. These groups are capable of covalently reacting with hydroxyl under physiological, or near-physiological conditions.

According to a further embodiment of the invention, each polypeptide subunit may be covalently bound to the activated polymer via a carbohydrate comprised within the polypeptide subunit, which carbohydrate has been previously chemically modified to comprise at least one aldehyde group. One of ordinary skill in the art is aware of how to convert a carbohydrate into an aldehyde, for example by treatment with mild (about 10 mM) sodium periodate. In eukaryotic cells, many polypeptides undergo posttranslational modifications involving glycosylation, i.e. functionalization of the expressed polypeptide with carbohydrate, the form of which may in some instances be quite complex. A non-covalently associated polypeptide multimer may be the result of expressing a recombinant polypeptide in a eukaryotic host expression system, for example a yeast or Chinese hamster ovary cell ("CHO") system, and may therefore bear such a glycosylation pattern. Such polypeptide multimers comprising glycosylated polypeptide subunits are also susceptible of the inventive use, as an activated polymer comprising a free amino group will react with a carbohydrate which has been at least partially converted into aldehyde to form a stable Schiff base which can then be converted to a stable secondary amine via reductive amination. Of course, a polypeptide subunit having undergone some degree of posttranslational modification need not be covalently bound to the activated polymer via its carbohydrate groups which have been refunctionalized as aldehydes; a coupling to activated polymer via any of the other chemistries mentioned herein above, i.e. directly between groups belonging to the polypeptide subunit's amino acids and the activated polymer, is also possible. As such, with posttranslationally modified polypeptides, coupling of activated polymer via an aldehyde-functionalized carbohydrate merely represents an additional mode of coupling within the use of the invention.

According to the invention, the activated polymer may be an activated polyethylene glycol ("PEG"). Activated PEG may take many commercially available forms, for example mPEG-SPA (mPEG-Succinimidyl Propionate), mPEG-SBA (mPEG-Succinimidyl Butanoate), mPEG-SMB (mPEG-Succinimidyl alpha-methylbutanoate), mPEG2-NHS (mPEG2-N-hydroxysuccinimide), mPEG-OPTE (mPEG-thioester), mPEG-CM-HBA-NHS (mPEG-carboxymethyl-3-hydroxybutanoic acid-N-hydroxysuccinate), mPEG-ACET (mPEG-Acetaldehyde diethyl acetal), mPEG2-Acetaldehyde (equivalent to mPEG2-diethyl acetal), mPEG-Propionaldehyde, mPEG2- Propionaldehyde, mPEG-Butyraldehyde, mPEG2-Butyraldehyde, mPEG-Ketones, mPEG-MAL (mPEG-Maleimide), mPEG2-MAL (mPEG2-Maleimide) and mPEG-Thiols (all of these polymers being commercially available from Nektar Therapeutics, San Carlos, CA, US). All are especially preferred as activated polymer within this embodiment of the invention provided a complement chemical group exists within the polypeptide subunit of the non-covalently polypeptide multimer by which covalent coupling may take place.

According to the invention, the activated polymer may be an activated polydextran or an activated poly-L-lysine.

According to a further embodiment of the invention, the activated polymer may be attached to the polypeptide subunit by means of non-covalent interactions which, under physiological conditions, typically exhibit close to the strength of a covalent chemical bond. An example of such strong non-covalent attachment may be the high affinity interaction of biotin to avidin or streptavidin. As is generally known, biotin and avidin, or biotin and streptavidin exhibit such high binding affinity for one another that their complex remains associated under typical physiological conditions. In this case, individual polypeptide subunits within the non-covalently associated polypeptide multimer must be functionalized with one member of the intended non-covalent complex, while the activated polymer must be functionalized with the other member of this complex such that, when the functionalized activated polymer is brought into contact with a respective functionalized polypeptide subunit, the strong non-covalent interaction between said two members will result, in effect, to each polypeptide subunit within the polypeptide multimer being bound to at least one molecule of activated polymer.

The invention will now be described in further detail by way of the following figures and examples.

### Summary of the figures:

- Fig. 1: Schematic representation of the inventive use
- Fig. 2: Elution profile of immobilized metal affinity chromatography (IMAC)-purified scFv polypeptide showing peak containing both monomeric and dimeric scFv
- Fig. 3: Elution profile resulting from size exclusion chromatography ("SEC") of the elution fraction containing the polypeptide peak shown in Fig. 2
- Fig. 4: SDS-PAGE analysis of scFv polypeptide fractions obtained from the SEC-and IMAC-analyses shown in Figs. 2 and 3
- Fig. 5: Independent, superimposed SEC elution profiles of monomeric and dimeric scFv polypeptides following independent PEGylation of each of these polypeptide fractions

The invention will now be described in further detail by way of examples.

### Example 1: Schematic illustration of the invention

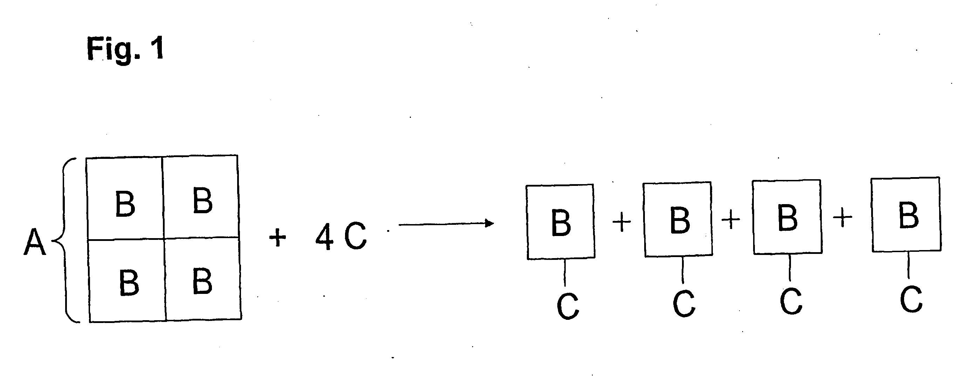

Fig. 1 shows in general form a schematic representation of the use according to the invention in which the letter A represents a non-covalently associated polypeptide multimer, the letter B represents a polypeptide subunit within the non-covalently associated polypeptide multimer A (for example, a respective polypeptide subunit B may be an scFv polypeptide), and the letter C represents one molecule of activated polymer.

Fig. 1 depicts the use of the invention in generic form, showing a scenario in which the non-covalently associated polypeptide multimer A is made up of four polypeptide subunits B. Each polypeptide subunit B is non-covalently associated to at least one other polypeptide subunit B such that the resulting non-covalently associated polypeptide multimer A is held together entirely by non-covalent interactions between its constituent polypeptide subunits B, i.e. no polypeptide subunit B is connected to any other polypeptide subunit B by a covalent chemical bond. It is assumed that each of the polypeptide subunits B comprises a chemical group capable of forming a covalent bond with at least one molecule of activated polymer C. The non-covalently associated polypeptide multimer A is reacted with at least four molecules of activated polymer C under conditions amenable to the formation of a covalent chemical bond between at least one molecule of activated polymer C and each of the polypeptide subunits B. The result of this reaction is that the individual polypeptide subunits B making up the non-covalently associated polypeptide multimer A are separated from one another to yield four individual polypeptide subunits B, each of which is covalently bound to at least one activated polymer C. Normally, each polypeptide subunit B will be identical, having resulted, for example, from recombinant expression from a host cell. However, it is possible that a non-covalently associated polypeptide multimer A may be composed, say, of polypeptide subunits B, B', B", etc., said subunits B, B', B", etc. differing from one another, as for example may be the case for incompletely expressed variants of a desired recombinant polypeptide. Regardless of whether the non-covalently associated polypeptide multimer A is composed of identical or non-identical polypeptide subunits B, said polypeptide subunits B may be separated from one another according to the use of the invention as long as they can form a covalent bond with at least one molecule of activated polymer. As Fig. 1 clearly illustrates, the use of the invention provides an efficient way of breaking up undesired polypeptide multimers, into multiple, homogeneous, desired polypeptide monomers, each desired polypeptide monomer being covalently bound to at least one molecule of polymer. In this way, polypeptide which would otherwise remain unresolvable in monomeric form can be resolved, increasing the overall yield of this monomeric polypeptide as bound to polymer.

### Example 2: Production and purification of an scFv polypeptide

An scFv polypeptide (i.e. a single polypeptide chain containing VH and VL antibody regions connected by a polypeptide linker) was expressed in *E.coli* BL21 DE3 transfected with a pBAD vector (Xoma) with kanamycin resistance, the pBAD vector encoding the desired scFv. The expression culture was incubated in LB medium containing 50 µg/mL kanamycin at 300 rpm and 37°C for 12 hours. Gene expression was induced by adding L-Arabinose to a total concentration of 0.08% (w/v), followed by further stirring at 300 rpm for 15 hours at 30°C.

Cells were then harvested by centrifugation at 10,000 x g for 15 min and were resuspended in a total of 900 mL 1X PBS. The scFv protein was extracted by 6 freeze-thaw cycles. Finally, the suspensions were centrifuged at 16,000 x g and 4°C for 15 min. The cleared supernatants were then used as crude periplasmic preparation.

Generally, the crude scFv protein was purified in a two step purification process including immobilized metal affinity chromatography (IMAC) and gelfiltration. An Äkta FPLC System (Pharmacia) and Unicorn Software were used for chromatography. All chemicals were of research grade and purchased from Sigma (Deisenhofen) or Merck (Darmstadt).

IMAC was performed using an NiNTA column (Qiagen) loaded with NiSO₄ according to the manufacturer's protocol. The column was equilibrated with buffer A (20 mM NaPP pH 7.5, 0.4 M NaCl, 10 mM imidazol) and the periplasmic preparation (500 ml), containing 10 mM imidazol was applied to the column (5 ml) at a flow rate of 3 ml/min. The column was washed with buffer A to remove unbound sample. Bound protein was eluted using 100% buffer B (20 mM NaPP pH 7.5, 0.4 M NaCl, 0.5 M Imidazol). Eluted protein fractions from the step using 100% buffer B were pooled for further purification.

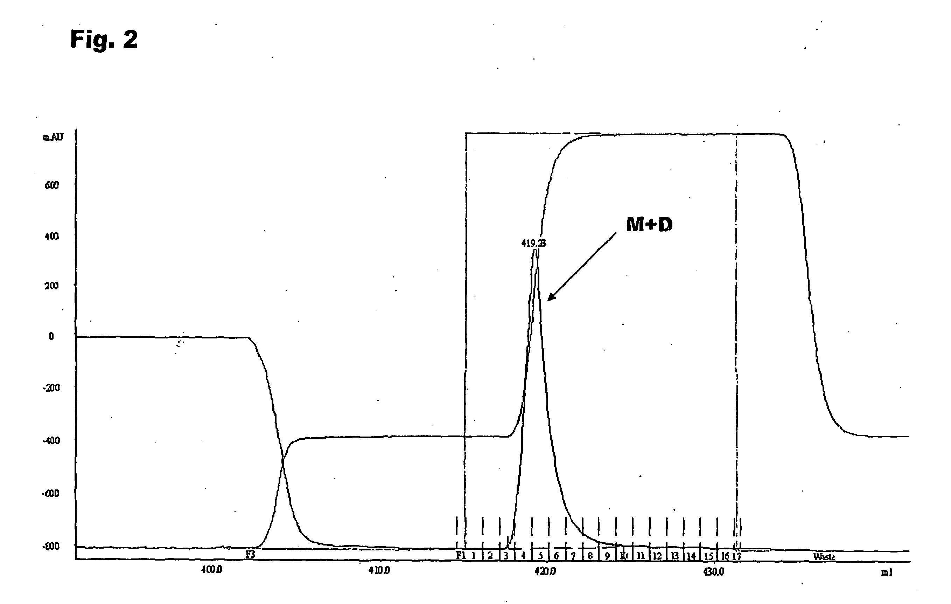

Results are shown iri Fig. 2, with the polypeptide peak eluting at approximately 420 ml. "M+D" indicates that this peak results from both monomeric as well as (homo)dimeric forms of the scFv. These two forms together form one peak in Fig. 2 because IMAC does not differentiate between polypeptides of different molecular weight but rather binds histidine-tagged proteins of any type.

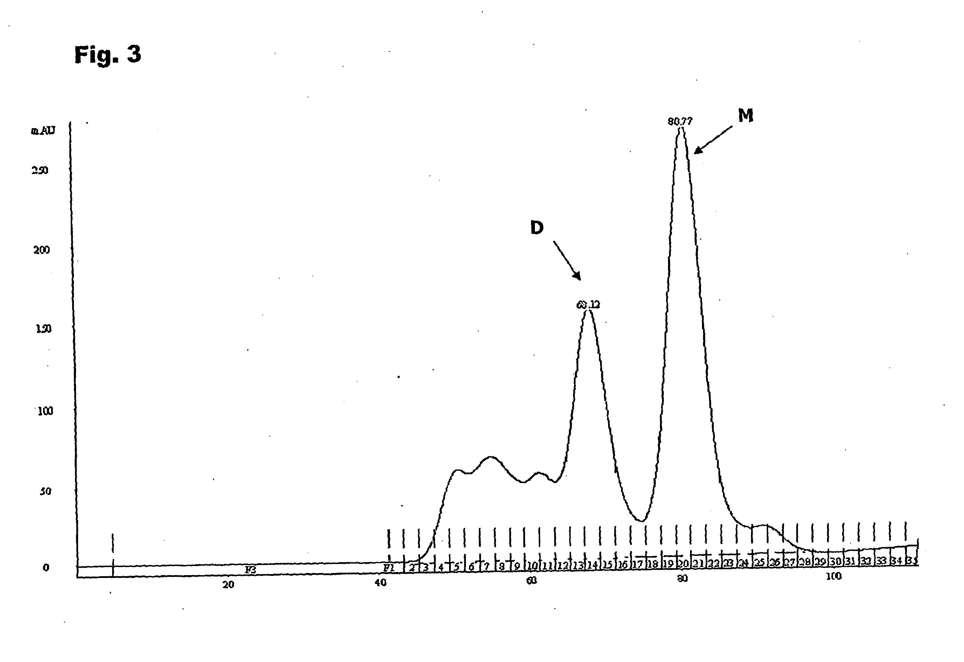

The polypeptide contained within the "M+D" elution peak of Fig. 2 was then subjected to gelfiltration chromatography (i.e. SEC) at a flow rate of 1 ml/min on a Superdex 200 HiPrep column (Pharmacia) or Sephadex 400 column equilibrated with 20 mM Tris pH 7.2, 250 mM NaCl, 5% v/v glycerol, 2 mM DTT. The column was previously calibrated for molecular weight determination (molecular weight marker kit, Sigma MW GF-200). Fig. 3 shows the results from the Superdex 200 HiPrep SEC column. Two main polypeptide peaks are observed, one at an elution volume of approximately 68 ml and another at an elution volume of approximately 80 ml. The former, indicated in Fig. 3 as "D", corresponds to the scFv polypeptide in dimeric form with a molecular weight of about 54 kD (i.e. a diabody-like structure in which two identical scFv polypeptides are linearly associated head-to-tail such that the VH of one scFv chain associates intermolecularly with the VL of another scFv chain), whereas the latter, indicated in Fig. 3 as "M", corresponds to the scFv polypeptide in monomeric form with a molecular weight of about 27 kD (i.e. an scFv in which the VH and VL of a single scFv chain associate intramolecularly with one another).

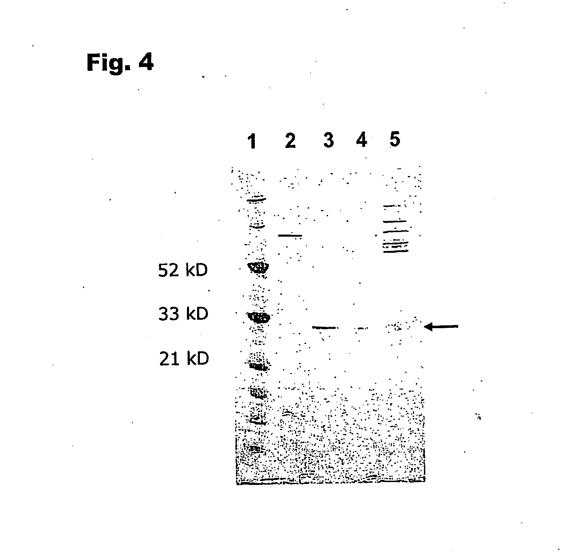

In order to confirm that the "D"-peak in Fig. 3 is in fact due to the non-covalently associated dimeric form of the polypeptide giving rise to the "M"-peak in Fig. 3, denaturing polyacrylamide gel electrophoresis (SDS-PAGE) was performed on the protein fractions obtained from gelfiltration chromatography described above. SDS-PAGE under reducing conditions was performed with precast 4-12% Bis Tris gels (Invitrogen). Sample preparation and application were according to the manufacturer's protocol. The molecular weight was determined with MultiMark protein standard (Invitrogen). The gel was stained with colloidal Coomassie (Invitrogen protocol). The results are shown in Fig. 4.

Lane 1 of Fig. 4 shows a molecular weight ladder. Lane 2 of Fig. 4 is the gelfiltration elution fraction containing the high molecular weight aggregates which eluted prior to the peak at approximately 68 ml, i.e. at elution volumes of about 48 ml to about 62 ml. Lane 3 of Fig. 4 shows the gelfiltration elution peak at about 68 ml attributed to the scFv dimer. Lane 4 of Fig. 4 shows the gelfiltration elution peak at about 80 ml attributed to the scFv monomer. Lane 5 of Fig. 4 shows the IMAC eluate containing all protein products obtained from the lysed *E. coli* cells. The horizontal arrow to the immediate right of the gel in Fig. 4 indicates the position of the scFv polypeptide monomer at about 27 kD.

Although the protein products of lanes 3 and 4 of Fig. 4 eluted from the gelfiltration column at volumes corresponding to, respectively, 54 kD and 27 kD, under denaturing and reducing gel conditions, these two protein products run identically on reducing SDS-PAGE, corresponding to the molecular weight of the scFv polypeptide monomer, 27 kD. This indicates that the scFv product obtained and purified from the cell lysate is in fact present in two forms, namely as a monomer scFv as well as a dimer scFv, dimeric scFv polypeptide being separated into constituent monomers under the denaturing and reducing conditions of PAGE.

### Example 3: PEGylation of the scFv product

ScFv monomeric and dimeric polypeptide fractions separated by gelfiltration chromatography were coupled to 40 kD polyethylene glycol maleimide ("PEG-MAL 40") in independent coupling reactions. The PEG-MAL used was a branched PEG bearing two chains of 20 kD molecular weight each, i.e. mPEG2-MAL. The scFv polypeptides had been engineered to contain a free cysteine residue at the C-terminus such that the scFv:PEG-MAL 40 coupling ratio could be controlled at 1:1. The following procedure was performed independently for the monomeric scFv and for the dimeric scFv.

The buffer used for coupling contained 50 mM Tris, 5 vol% glycerol, 2 mM DTT adjusted to pH 7.2. This formulation stabilized the proteins and kept them in solution, preventing unwanted aggregation and precipitation. The DTT was included to cleave any unwanted cysteine disulfide bond at the C-terminus of the scFv polypeptide, ensuring the presence of a free thiol group which can covalently react with the maleimide group in PEG-MAL 40. Prior to PEGylation, DTT was removed by size exclusion chromatography using Sephadex G25 Medium (Amersham Biosciences). Here, the loading volume was kept below 10% of the column volume to avoid breakthrough of free DTT. The column was equilibrated with a buffer containing 400 mM NaCl, 500 mM imidazole, 20 mM phosphate adjusted to pH 7.2. The same buffer was used for elution.

The eluate was collected in 90 µl fractions into a 96-well plate made of low protein-binding polypropylene. Protein-containing fractions were detected by transfer of 5 µl of each well into a 96-well plate, each well of which containing a 4:1 mixture of PBS and Bradford reagent (BioRad). Proteins cause this mixture to change color from light brown to blue and optical absorption at 595 nm was measured on a Tecan Spectrafluor Plus plate reader to confirm the presence of proteinaceous material. Protein-containing fractions were pooled and protein concentrations were determined by measuring absorption at 280 nm and using the molar absorption coefficient.

PEG-MAL 40 was weighed into two reaction tubes with round bottoms, one tube for the reaction of monomeric scFv polypeptide with PEG-MAL 40, and the other tube for the reaction of dimeric scFv polypeptide with PEG-MAL 40. A molecular excess of 5 PEG molecules to 1 scFv polypeptide molecule was calculated with a minimum of 2.5 mg PEG Maleimide per ml final volume. Polypeptide solutions containing the scFv monomer and scFv dimer were transferred into two separate tubes and PEG was dissolved by gentle mixing with a pipette. Incubation was performed on a Dynal flip-over rotation mixer in the dark for two hours at room temperature or over night at 5°C.

### Example 4: Comparison of the scFv monomer and dimer following independent coupling to PEG-MAL 40

The scFv-PEG conjugates were purified by cation exchange chromatography to remove free PEG and unconjugated polypeptide (results not shown), and the bioactivity of the final products was confirmed. The final purified scFv-PEG-MAL 40 conjugates resulting from independent coupling of the monomeric and dimeric scFv polypeptides were tested for purity on SDS-PAGE and both PEGylated monomer and dimer migrated at a molecular weight of about 100 kD, the expected size of the product by this detection method due to the non-globular, i.e. linear characteristics of PEG, which cause PEG to run differently on SDS-PAGE than a protein of corresponding molecular weight (results not shown).

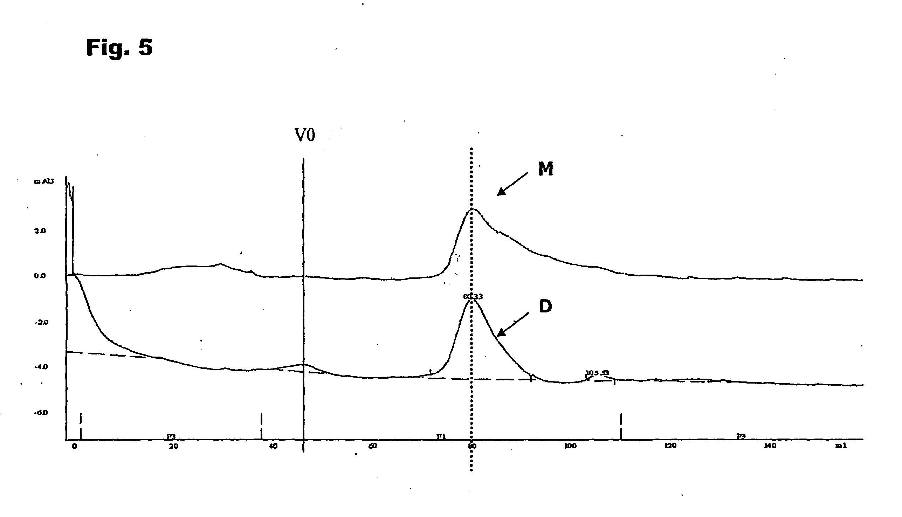

In addition, the reaction products resulting from independent coupling of monomeric and dimeric scFv with PEG-MAL 40 were analyzed by SEC. The results of this comparative analysis are shown in Fig. 5, in which "V0" indicates the void volume of the size exclusion column, "M" indicates the protein peak corresponding to PEGylated scFv monomer polypeptide, and "D" indicates the protein peak corresponding to PEGylated scFv dimer polypeptide. As can be clearly seen in Fig. 5, both types of PEGylated scFv polypeptide exhibit identical column retention times (indicated at the vertical dashed line), meaning that PEGylation of an scFv polypeptide in dimeric form results in the same product as PEGylation of the corresponding scFv polypeptide in monomeric form, namely the PEGylated scFv monomer. This result was further confirmed by cation exchange chromatography analysis; the ionic strength necessary for elution was identical for both PEGylated scFv monomer and PEGylated scFv dimer (results not shown).

## Claims

1. Use of an activated polymer to separate a non-covalently associated polypeptide multimer comprising multiple polypeptide subunits into multiple polypeptide subunits, wherein
- the activated polymer comprises a chemical moiety by which the polymer can be covalently bound to the polypeptide subunit in the non-covalently associated polypeptide multimer,
- the activated polymer is an activated polyethylene glycol, an activated polydextran, or an activated poly-L-lysine, and
- each of the polypeptide subunits comprises a single polypeptide chain; and/or a group of at least two single polypeptide chains, wherein the at least two single polypeptide chains are covalently bound to one another and wherein at least one of the polypeptide subunits comprises a single chain antibody comprising at least one antibody variable region.

2. The use of claim 1, wherein the activated polymer has a molecular weight of at least 3,000 g/mol and comprises from 25 to 70 wt.% polar atoms, wherein polar atoms are atoms which enter into hydrogen-bonded interactions with water molecules in aqueous solution.

3. The use of claim 1 or 2, wherein each of the multiple polypeptide subunits in its separated form is bound to the activated polymer.

4. The use of claim 3, wherein each of the multiple polypeptide subunits in its separated form is covalently bound to the activated polymer.

5. The use of any of the preceding claims, wherein the single chain antibody comprises one or two antibody variable region(s).

6. The use of any of the preceding claims, wherein each of the polypeptide subunits is covalently bound to the activated polymer via an amino group, a sulfhydryl group, a carboxyl group, a hydroxyl group or an aldehyde group comprised within/on the polypeptide subunit.

7. The use of claim 6, wherein
• the activated polymer which is capable of forming a covalent chemical bond with an amino group comprised within the polypeptide subunit comprises a hydroxysuccinimidyl group, a carboxyl group, an epoxide group, a keto group or an aldehyde group;
• the activated polymer which is capable of forming a covalent chemical bond with a sulfhydryl group comprised within the polypeptide subunit comprises a maleimide group, a vinyl sulfone group or a sulfhydryl group;
• the activated polymer which is capable of forming a covalent chemical bond with a carboxyl group comprised within the polypeptide subunit comprises an amino group or a hydroxyl group; and/or
• the activated polymer which is capable of forming a covalent chemical bond with a hydroxyl group comprised within the polypeptide subunit comprises a carboxyl group, an aldehyde group or a keto group.

8. The use of any of claims 3-7, wherein each polypeptide subunit is covalently bound to the activated polymer via a carbohydrate comprised within the polypeptide subunit, which carbohydrate has been chemically modified to comprise at least one aldehyde group.

9. The use of claim 8, wherein the activated polymer which is capable of forming a covalent chemical bond with the aldehyde group-comprising carbohydrate comprises an amino group or a hydrazide group.

10. The use of claim 9, wherein the covalent bond between the aldehyde and the amino group or hydrazide group is stabilized by reductive amination.

11. The use of claim 1, wherein the activated polyethylene glycol is chosen from the group consisting of mPEG-SPA (mPEG-Succinimidyl Propionate), mPEG-SBA (mPEG-Succinimidyl Butanoate), mPEG-SMB (mPEG-Succinimidyl alpha-methylbutanoate), mPEG2-NHS (mPEG2-N-hydroxysuccinimide), mPEG-OPTE (mPEG-thioester), mPEG-CM-HBA-NHS (mPEG-carboxymethyl-3-hydroxybutanoic acid-N-hydroxysuccinate), mPEG-ACET (mPEG- Acetaldehyde diethyl acetal), mPEG2- Acetaldehyde (equivalent to mPEG2-diethyl acetal), mPEG-Propionaldehyde, mPEG2- Propionaldehyde, mPEG-Butyraldehyde, mPEG2-Butyraldehyde, mPEG-Ketones, mPEG-MAL (mPEG-Maleimide), mPEG2-MAL (mPEG2-Maleimide) and mPEG-Thiols.

12. The use of any of the preceding claims, wherein the activated polymer has a molecular weight of 3,500 g/mol, 5,000 g/mol, 20,000 g/mol, or 40,000 g/mol.

13. The use of claim 11 or claim 12, wherein the mPEG-MAL is mPEG-MAL or mPEG2-MAL having a molecular weight of 40,000 g/mol.

14. The use of any of the preceding claims, wherein the activated polymer comprises
• from 27 to 60 wt.% polar atoms, in particular 32 to 45 wt.% polar atoms, from 35 to 38 wt.% polar atoms, or
• from 36 to 37 wt.% polar atoms;
• from 27 to 28 wt.% polar atoms;
• from 48 to 50 wt.% polar atoms; or
• from 54 to 56 wt.% polar atoms,
wherein polar atoms are atoms which enter into hydrogen-bonded interactions with water molecules in aqueous solution.

## Patentansprüche

1. Verwendung eines aktivierten Polymers, um ein nicht-kovalent assoziierte Polypeptidmultimer, umfassend mehrere Polypeptiduntereinheiten, in mehrere Polypeptiduntereinheiten zu trennen, wobei
• das aktivierte Polymer eine chemischen Rest umfasst, durch welche das Polymer kovalent an die Polypeptiduntereinheit in dem nicht-kovalent assoziierten Polypeptidmultimer gebunden werden kann,
• das aktivierte Polymer ein aktiviertes Polyethylenglykol, ein aktiviertes Polydextran oder ein aktiviertes Poly-L-Lysin ist und
• jede der Polypeptiduntereinheiten eine einzelne Polypeptidkette und/oder eine Gruppe von mindestens zwei einzelnen Polypeptidketten umfasst, wobei die mindestens zwei einzelnen Polypeptidketten kovalent aneinander gebunden sind und wobei mindestens eine der Polypeptiduntereinheiten einen einzelkettigen Antikörper umfasst, der mindestens eine variable Region eines Antikörpers umfasst.

2. Verwendung nach Anspruch 1, wobei das aktivierte Polymer ein Molekulargewicht von mindestens 3.000 g/mol hat und 25 bis 70 Gew.-% polare Atome umfasst, wobei polare Atome Atome sind, die Wasserstoffbrückenwechselwirkungen eingehen mit Wassermolekülen in wässriger Lösung.

3. Verwendung nach Anspruch 1 oder 2, wobei jede der mehreren Polypeptiduntereinheiten in ihrer getrennten Form an das aktivierte Polymer gebunden ist.

4. Verwendung nach Anspruch 3, wobei jede der mehreren Polypeptiduntereinheiten in ihrer getrennten Form kovalent an das aktivierte Polymer gebunden ist.

5. Verwendung nach einem der vorherigen Ansprüche, wobei der einzelkettige Antikörper eine oder zwei variable Region(en) eines Antikörpers umfasst.

6. Verwendung nach einem der vorherigen Ansprüche, wobei jede der Polypeptiduntereinheiten kovalent gebunden ist an das aktivierte Polymer über eine Aminogruppe, eine Sulfhydrylgruppe, eine Carboxylgruppe, eine Hydroxylgruppe oder eine Aldehydgruppe, enthalten in/auf der Polypeptiduntereinheit.

7. Verwendung nach Anspruch 6, wobei
• das aktivierte Polymer, welches geeignet ist zur Bildung einer kovalenten chemischen Bindung mit einer Aminogruppe enthalten in der Polypeptiduntereinheit, eine Hydroxysuccinimidylgruppe, eine Carboxylgruppe, eine Epoxidgruppe, eine Ketogruppe oder eine Aldehydgruppe umfasst;
• das aktivierte Polymer, welches geeignet ist zur Bildung einer kovalenten chemischen Bindung mit einer Sulfhydrylgruppe enthalten in der Polypeptiduntereinheit, eine Maleimidgruppe, eine Vinylsulfongruppe oder eine Sulfhydrylgruppe umfasst;
• das aktivierte Polymer, welches geeignet ist zur Bildung einer kovalenten chemischen Bindung mit einer Carboxylgruppe enthalten in der Polypeptiduntereinheit, eine Aminogruppe oder eine Hydroxylgruppe umfasst; und/oder
• das aktivierte Polymer, welches geeignet ist zur Bildung einer kovalenten chemischen Bindung mit einer Hydroxylgruppe enthalten in der Polypeptiduntereinheit, eine Carboxylgruppe, eine Aldehydgruppe oder eine Ketogruppe umfasst.

8. Verwendung nach einem der Ansprüche 3-7, wobei jede Polypeptiduntereinheit kovalent gebunden ist an das aktivierte Polymer über ein Kohlenhydrat, das enthalten ist in der Polypeptiduntereinheit, wobei das Kohlenhydrat chemisch verändert worden ist, um mindestens eine Aldehydgruppe zu umfassen.

9. Verwendung nach Anspruch 8, wobei das aktivierte Polymer, welches geeignet ist zur Bildung einer kovalenten chemischen Bindung mit dem Aldehydgruppe(n)-umfassenden Kohlenhydrat in der Lage ist, eine Aminogruppe oder eine Hydrazidgruppe umfasst.

10. Verwendung nach Anspruch 9, wobei die kovalente Bindung zwischen der Aldehyd- und der Aminogruppe oder Hydrazidgruppe stabilisiert ist durch reduktive Aminierung.

11. Verwendung nach Anspruch 1, wobei das aktivierte Polyethylenglykol ausgewählt ist aus der Gruppe bestehend aus mPEG-SPA (mPEG-Succinimidylpropionat), mPEG-SBA (mPEG-Succinimidylbutanoat), mPEG-SMB (mPEG-Succinimidylalphamethylbutanoat), mPEG2-NHS (mPEG2-N-Hydroxysuccinimid), mPEG-OPTE (mPEG-Thioester), mPEG-CM-HBA-NHS (mPEG-Carboxymethyl-3-hydroxybutansäure-N-hydroxysuccinat), mPEG-ACET (mPEG-Acetaldehyddiethylacetal), mPEG2-Acetaldehyd (äquivalent zu mPEG2-Diethylacetal), mPEG-Propionaldehyd, mPEG2-Propionaldehyd, mPEG-Butyraldehyd, mPEG2-Butyraldehyd, mPEG-Ketonen, mPEG-MAL (mPEG-Maleimid), mPEG2-MAL (mPEG2-Maleimid) und mPEG-Thiolen.

12. Verwendung nach einem der vorherigen Ansprüche, wobei das aktivierte Polymer ein Molekulargewicht von 3.500 g/mol, 5.000 g/mol, 20.000 g/mol oder 40.000 g/mol hat.

13. Verwendung nach Anspruch 11 oder Anspruch 12, wobei das mPEG-MAL ein mPEG-MAL oder mPEG2-MAL ist, mit einem Molekulargewicht von 40.000 g/mol.

14. Verwendung nach einem der vorherigen Ansprüche, wobei das aktivierte Polymer
• von 27 bis 60 Gew.-% polare Atome, insbesondere 32 bis 45 Gew.-% polare Atome, von 35 bis 38 Gew.-% polare Atome oder
• von 36 bis 37 Gew.-% polare Atome;
• von 27 bis 28 Gew.-% polare Atome;
• von 48 bis 50 Gew.-% polare Atome; oder
• von 54 bis 56 Gew.-% polare Atome,
umfasst,
wobei polare Atomen Atome sind, die mit Wassermolekülen in wässriger Lösung Wasserstoffbrückenwechselwirkungen eingehen.

## Revendications

1. Utilisation d'un polymère activé pour séparer en multiples sous-unités polypeptidiques un multimère polypeptidique associé de façon non covalente comprenant de multiples sous-unités polypeptidiques, dans laquelle
- le polymère activé comprend un fragment chimique par lequel le polymère peut être lié de façon covalente à la sous-unité polypeptidique dans le multimère polypeptidique associé de façon non covalente,
- le polymère activé est un polyéthylène glycol activé, un polydextrane activé ou une poly-L-lysine activée, et
- chacune des sous-unités polypeptidiques comprend une chaîne polypeptidique simple ; et/ou un groupe d'au moins deux chaînes polypeptidiques simples, dans lesquelles les au moins deux chaînes polypeptidiques simples sont liées l'une à l'autre de façon covalente et au moins une desdites sous-unités polypeptidiques comprend un anticorps monocaténaire contenant au moins une région variable d'anticorps.

2. Utilisation selon la revendication 1, dans laquelle le polymère activé a un poids moléculaire d'au moins 3 000 g/mol et comprend de 25 à 70 % en poids d'atomes polaires, dans laquelle les atomes polaires sont des atomes qui s'engagent dans des interactions impliquant des liaisons hydrogène avec des molécules d'eau en solution aqueuse.

3. Utilisation selon les revendications 1 ou 2, dans laquelle chacune des multiples sous-unités polypeptidiques sous sa forme séparée est liée au polymère activé.

4. Utilisation selon la revendication 3, dans laquelle chacune des multiples sous-unités polypeptidiques sous sa forme séparée est liée de façon covalente au polymère activé.

5. Utilisation selon l'une quelconque des revendications précédentes, dans laquelle l'anticorps monocaténaire comprend une ou deux régions variables d'anticorps.

6. Utilisation selon l'une quelconque des revendications précédentes, dans laquelle chacune des sous-unités polypeptidiques est liée de façon covalente au polymère activé par l'entremise d'un groupe amino, un groupe sulfhydryle, un groupe carboxyle, un groupe hydroxyle ou un groupe aldéhyde présent dans/sur la sous-unité polypeptidique.

7. Utilisation selon la revendication 6, dans laquelle
• le polymère activé qui est capable de former une liaison chimique covalente avec un groupe amino présent dans la sous-unité polypeptidique comprend un groupe hydroxysuccinimidyle, un groupe carboxyle, un groupe époxyde, un groupe céto ou un groupe aldéhyde ;
• le polymère activé qui est capable de former une liaison chimique covalente avec un groupe sulfhydryle présent dans la sous-unité polypeptidique comprend un groupe maléimide, un groupe vinylsulfone, ou un groupe sulfhydryle ;
• le polymère activé qui est capable de former une liaison chimique covalente avec un groupe carboxyle présent dans la sous-unité polypeptidique comprend un groupe amino ou un groupe hydroxyle ; et/ou
• le polymère activé qui est capable de former une liaison chimique covalente avec un groupe hydroxyle présent dans la sous-unité polypeptidique comprend un groupe carboxyle, un groupe aldéhyde ou un groupe céto.

8. Utilisation selon l'une quelconque des revendications 3 à 7, dans laquelle chaque sous-unité polypeptidique est liée de façon covalente au polymère activé par l'entremise d'un glucide présent dans la sous-unité polypeptidique, lequel glucide a été chimiquement modifié pour comprendre au moins un groupe aldéhyde.

9. Utilisation selon la revendication 8, dans laquelle le polymère activé qui est capable de former une liaison chimique covalente avec le glucide comprenant un groupe aldéhyde comprend un groupe amino ou un groupe hydrazide.

10. Utilisation selon la revendication 9, dans laquelle la liaison covalente entre l'aldéhyde et le group amino ou le groupe hydrazide est stabilisée par amination réductive.

11. Utilisation selon la revendication 1, dans laquelle le polyéthylène glycol activé est choisi dans le groupe constitué par mPEG-SPA (mPEG-propionate de succinimidyle), mPEG-SBA (mPEG-butanoate de succinimidyle), mPEG-SMB (mPEG-alpha-méthylbutanoate de succinimidyle), mPEG2-NHS (mPEG2-N-hydroxysuccinimide), mPEG-OPTE (mPEG-thioester), mPEG-CM-HBA-NHS (mPEG-N-hydroxysuccinate d'acide carboxyméthyl-3-hydroxybutanoïque), mPEG-ACET (mPEG-acétaldéhyde diéthylacétal), mPEG2-acétaldéhyde (équivalent de mPEG2-diéthylacétal), mPEG-propionaldéhyde, mPEG2-propionaldéhyde, mPEG-butyraldéhyde, mPEG2-butyraldéhyde, mPEG-cétones, mPEG-MAL (mPEG-maléimide), mPEG2-MAL (mPEG2-maléimide), et mPEG-thiols.

12. Utilisation selon l'une quelconque des revendications précédentes, dans laquelle le polymère activé a un poids moléculaire de 3 500 g/mol, 5 000 g/mol, 20 000 g/mol ou 40 000 g/mol.

13. Utilisation selon la revendication 11 ou la revendication 12, dans laquelle le mPEG-MAL est un mPEG-MAL ou mPEG2-MAL ayant un poids moléculaire de 40 000 g/mol.

14. Utilisation selon l'une des revendications précédentes, dans laquelle le polymère activé comprend
• de 27 à 60 % en poids d'atomes polaires, en particulier de 32 à 45 % en poids d'atomes polaires, de 35 à 38 % en poids d'atomes polaires, ou
• de 36 à 37 % en poids d'atomes polaires ;
• de 27 à 28 % en poids d'atomes polaires ;
• de 48 à 50 % en poids d'atomes polaires ; ou
• de 54 à 56 % en poids d'atomes polaires,
dans laquelle les atomes polaires sont des atomes qui s'engagent dans des interactions impliquant des liaisons hydrogène avec des molécules d'eau en solution aqueuse.
